# EUROPEAN PATENT SPECIFICATION

(11) **EP 1 534 162 B1**
(45) Date of publication and mention of the grant of the patent: **04.04.2012**
(21) Application number: 02768514.8
(22) Date of filing: 13.08.2002
(51) Int. Cl.: A61F 5/00

(54) **REMOTELY ADJUSTABLE GASTRIC BANDING DEVICE**
FERNVERSTELLBARE MAGENBANDVORRICHTUNG
DISPOSITIF DE CERCLAGE GASTRIQUE AJUSTABLE A DISTANCE

(43) Date of publication of application: 01.06.2005
(73) Proprietor: ALLERGAN, INC., Irvine CA 92612 (US)
(72) Inventor: COE, Frederick, L., Santa Barbara, CA 93103 (US)
(74) Representative: Williams, Aylsa
(86) International application number: PCT/US2002/025654
(87) International publication number: WO 2004/014245

(56) References cited:
- EP-A1- 0 876 808
- WO-A-01/47435
- WO-A-02/19953
- FR-A1- 2 783 153
- US-A- 5 226 429
- US-A- 6 067 991
- US-A- 6 102 922
- US-B1- 6 210 347
- US-B1- 6 432 040
- US-B1- 6 450 946

## Description

### BACKGROUND OF THE INVENTION

### Field of the Invention

The present invention relates generally to an apparatus for remotely adjusting the volume in the inflatable portion of a surgically implanted gastric band encircling the stomach. A method for treating morbid obesity utilizing a remotely adjustable gastric banding device is also disclosed herein for reference.

### Description of the Related Art

A belt-like gastric band for encircling the stomach to control morbid obesity is disclosed by Vincent in U.S. Pat. 5,601,604. The band comprises a belt that can be passed around the stomach and locked into an encircling position in order to create a stoma opening within the stomach. An adjustable portion of the band comprises an inflatable member which permits fine adjustment of the stoma opening after the stoma is created by locking the band in place.

The gastric banding procedure may involve placement of a calibrating apparatus in the stomach to position the stoma and size the pouch created above the stoma. The gastric band is fastened in position about the stomach to prevent slippage, usually by gastro-gastric sutures.

The stoma opening may be adjusted by injecting or withdrawing a fluid into or from an inflatable member, which is preferably coextensive with a portion of the inner stomach-contacting surface of the band. The means for injecting the fluid into the inflatable member usually comprises a fill port located beneath the skin that can be accessed extracorporeally by transdermal injection. Thus, following implantation, the gastric band can be adjusted to enlarge or reduce the stoma as required.

A potential disadvantage of prior art gastric bands is the difficulty in finely adjusting the stoma created by the implanted band. For example, the fill port located beneath the skin can be difficult to locate precisely. In addition, the fill procedure requires an invasive transdermal injection to adjust the band. Hence, repeated adjustments may be painful or worrisome to the patient. Moreover, exposure to x-rays may be required to facilitate location of the port. It would therefore be desirable to provide a band having an inflatable member that can be easily, precisely, and readily adjusted remotely, without the need to undergo an invasive procedure or radiographic exposure.

To address this problem, several prior art remote control gastric banding devices have been proposed. Klaiber et al. (U.S. Pat. 5,938,669 and EP 0876808) discloses a radio controlled gastric band adjusted by means of an electric pump and a balancing reservoir. Forsell (U.S. Pat. 6,210,347) discloses a remotely controlled and powered gastric band adjusted by a motorized mechanical or hydraulic means. WO 01/47435 discloses a heartburn and reflex disease treatment apparatus comprising an adjustable restriction device and a hydraulic operation means. Each of these proposed devices operates by pumping fluid to or from the gastric band. Unfortunately, because of their energy requirements, these devices pose problems for practical use. These devices are also not suitable for use with existing gastric banding systems, such as that disclosed by Vincent.

Recent developments in implantable drug delivery devices have shown that small, reliable, and energy-efficient implantable devices are feasible. Drug delivery devices currently exist in which drugs are administered periodically or continuously to a patient having an implanted device by applying pressure from a pressurized reservoir and opening an outlet valve to allow a pressure differential to cause a flow of the drug. For example, Malamud et al. (U.S. Patent 5,928,195) discloses a remotely controlled drug delivery device suitable for implantation in a body cavity. A pressurized gas chamber presses upon a drug storage chamber thereby administering a dose of the drug when a valve is remotely opened.

Similarly, Arzbaecher (U.S. Patent 5,607,418) discloses an implantable drug apparatus having nested deformable chambers with the outer chamber being pressurized. The pressure from the outer pressurized chamber forces the drug from a reservoir chamber into an inner dispensing chamber. A remotely controlled valve is used to administer a dose of the drug from the dispensing chamber. Further, Haller et al. (U.S. Patent 6,203,523) discloses an implantable drug infusion device having a flow regulating mechanism that permits the flow rate to be independent of reservoir pressure. Some of the tradeoffs between "passive" (pressurized reservoir-based) devices and "active" (pump-based) devices are discussed in Haller.

### OBJECTS OF THE INVENTION

The foregoing demonstrates a need for a practical, accurate and easy means of remotely adjusting an implanted gastric band.

It is therefore an object of the present invention to provide a practical, accurate and efficient means for remotely adjusting an implanted gastric band.

It is another an object of the present invention to remotely adjust an implanted gastric band having an inflatable member.

It is yet another object of the invention to provide a remote control means suitable for use with existing gastric banding devices and technology.

Still another an object of the present invention is to minimize device complexity for an implanted remotely adjustable gastric banding device to ensure maximum device longevity/durability, in light of the fact that repair would require additional surgery.

Various other objects, advantages and features of the present invention will become readily apparent from the ensuing detailed description and the novel features will be particularly pointed out in the appended claims.

### SUMMARY OF THE INVENTION

The present invention applies recent developments in implantable drug delivery device technology to the field of gastric banding.

The invention provides a gastric banding device for treatment of morbid obesity. The device has a gastric band suited for laparoscopic placement around the stomach of a patient to form an adjustable stoma opening. The gastric band has an inflatable chamber for adjusting the inner circumference of the band. The inflatable chamber is preferably substantially coextensive with an inner stomach-facing surface of the gastric band. The inflatable member does not wrinkle or fold when adjusted, thereby presenting a substantially smooth contour along the inner circumference. A fluid-filled pressurized reservoir provides a source of fluid to inflate the inflation chamber of the gastric band. First and second valves control the flow between the pressurized reservoir, the inflatable chamber, and an unpressurized or negatively pressurized outlet. A controller is used to control the valves, thereby regulating the volume change in the inflatable chamber to adjust the inner circumference of the band. The controller is remotely controllable from outside of the patient.

Other embodiments of the invention include a remote control for remotely transmitting control signals to the controller, a receiver for receiving control signals from the remote control, and a power source for providing power to the controller and the valves. The power source may be an induction coil. The power source may also be a battery or capacitor charged by a piezoelectric device which converts body motion into electrical energy.

In a method disclosed herein for reference, a remotely adjustable gastric banding system may be use for the treatment of obesity. The method comprises the steps of implanting a gastric band, preferably laparoscopically, around the stomach of the patient to create a stoma; remotely transmitting control signals from outside of the patient to a controller of the implanted gastric banding device; and actuating a first valve, between a pressurized reservoir and an inflatable chamber, and/or a second valve, between the inflatable chamber and an outlet, on the basis of the control signals received by the controller to increase or decrease the fluid volume in the inflatable chamber, thereby adjusting the inner circumference of the band to adjust the stoma.

### BRIEF DESCRIPTION OF THE DRAWINGS

The following detailed description given by way of example, but not intended to limit the invention solely to the specific embodiments described, may best be understood in conjunction with the accompanying drawings in which:
Figure 1 is a perspective view of a laparoscopically implantable gastric band, which may be used in the present invention, fastened in an encircling position and partially inflated;
Figure 2 is a side view of the gastric band shown in Figure 1; and
Figure 3 is a schematic diagram showing a remotely controlled fluid distribution system for a gastric band according to the present invention.

### DETAILED DESCRIPTION OF CERTAIN PREFERRED EMBODIMENTS

The present invention combines the implantable drug delivery device technology discussed above with gastric banding technology. The preferred embodiments of the apparatus according to the present invention will be described with reference to the accompanying drawings.

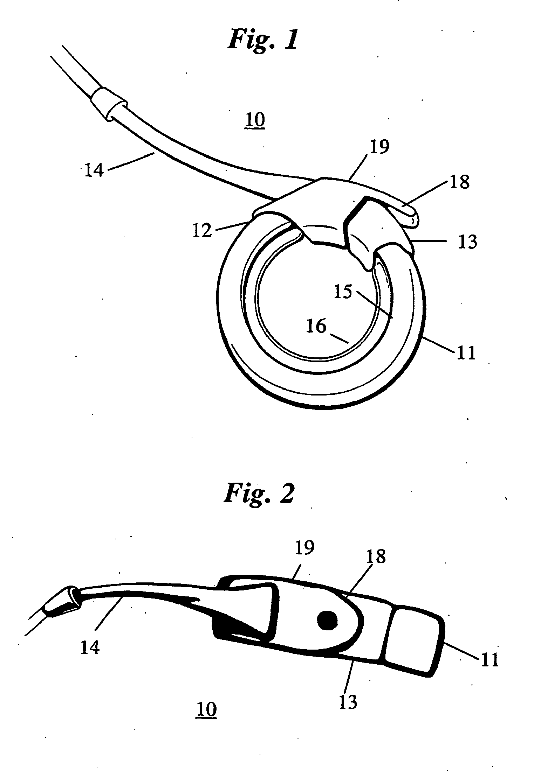

Referring to Figure 1, a gastric band for use with the present invention is disclosed in Vincent (U.S. Patent 5,601,604). This compatible gastric band, indicated as reference numeral **10,** has a body portion **11** with an inner stomach-facing surface **15.** The body portion **11** has a head end **12** and a tail end or "belt" **13.** A fill tube **14,** which is generally a tube having a single lumen coextensive therewith, is in fluid communication with an inflatable chamber **16** on the inner surface **15** of the band body **11.** Preferably, the inflatable portion **16** is substantially coextensive with the inner surface **15** of the body portion **11.** The central lumen of the fill tube **14** is in fluid communication with inflatable chamber **16.** The head end **12** of the body portion **11** has a "buckle" **19** through which the tail end of "belt" **13** is inserted and locked in place in use. Head end **12** may be provided with a pull tab **18** for use in locking the band in place about the stomach.

In use, the gastric band is placed in an encircling position around the stomach and locked in place as shown in Figure 2. (In Figure 2, the stomach is omitted for clarity.) This is accomplished by introducing the gastric band **10** through a laparoscopic cannula (not shown) in a patient's abdominal cavity. Laparoscopic placement consists of blunt dissection below the gastro-esophageal junction followed by placement of the band. The end of the fill tube **14** is passed through the dissected path around the upper stomach, and the tail end or belt **13** is passed through buckle **19,** so that the belt and buckle lock in place. A laparoscopic closure tool, such as that disclosed by Coe and Vincent in U.S. Pat. 5,658,298 may be used. Hence, with the gastric band affixed in an encircling position around the stomach, a new stoma (opening) is created within the stomach. After the band is secured in position, the size of the stoma may be adjusted by adding fluid to or withdrawing fluid from the inflatable member **16** to bring the stoma opening to the desired size. The inflatable member or chamber **16** is preferably coextensive with the inner stomach-facing surface **15** of the band between the head end **12** and the tail end **13.** The interior of the adjustable chamber **16** is in fluid communication with a fluid reservoir (not shown) by means of the central lumen of the fill tube **14,** as with prior art adjustable gastric bands. The inflatable member **16** is gradually inflated or deflated with saline or other biologically compatible fluid via the fluid reservoir such that the inflatable member **16** presses on and constricts the stomach wall or other tissue underlying the band. This results in the decrease or increase of the size of the stomach opening directly inside the encircling band.

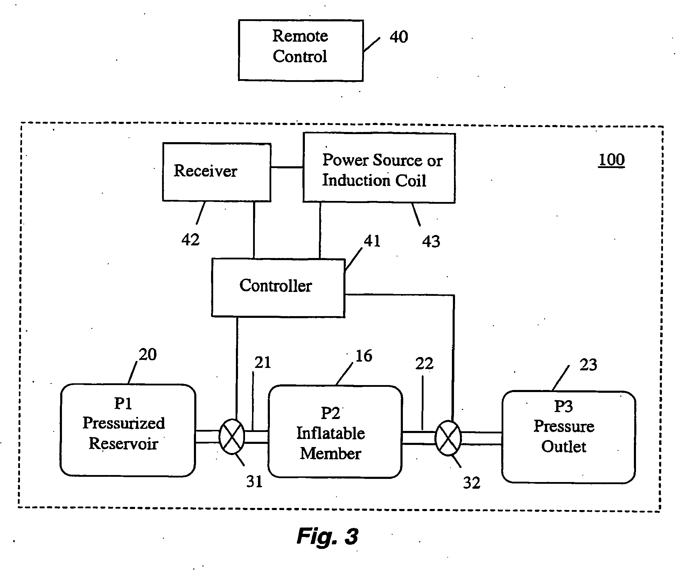

Figure 3 is a schematic diagram depicting a remotely adjustable gastric band **100** constructed in accordance with the present invention. In Figure 3, the pressure in the inflatable member **16** of the remote gastric banding system **100** is represented by the band inflation pressure P2. Pressure P2 is regulated by an inlet valve **31** and an outlet valve **32.** Pressurized reservoir **20,** having a pressure P1, is connected to the inflatable chamber **16** through inlet valve **31** and tube **21,** which corresponds to fill tube **14** in Figure 2. Pressurized reservoir **20** is analogous to the pressurized reservoirs discussed above in relation to implantable drug delivery devices. This reservoir may be connected to the fill tube **21** as shown, or it may be incorporated into the body **11** of the band itself, *e.g*. on the outer surface, opposite the inner stomach-facing surface **15,** and communicate directly with inflatable chamber **16** though inlet valve **21.** Inflatable member **16** is also connected to outlet **23,** having a pressure P3, through tube **22** and valve **32.** Outlet **23** may be either a separate waste reservoir as shown in Figure 3 or the peritoneal cavity of the patient's body. When outlet **23** is a waste reservoir, P3 may be negative. Where pressure outlet **23** is the patient's peritoneal cavity, P3 will be at ambient pressure within the body.

In the present invention, the pressure relationship between reservoir **20,** inflatable member **16** and outlet **23** is initially represented by the formula P1 > P2 > P3. Hence, valve **31** may be used to increase the pressure P2 up to a maximum pressure of P2=P1, thereby inflating inflatable member **16.** Similarly, valve **32** may be used to decrease the pressure P2 down to a minimum of P2=P3, thereby deflating inflatable member **16.** Thus, by actuating valves **31** and **32,** the fluid volume in the inflatable member **16** may be regulated, thereby adjusting the size of the stoma formed by the gastric band.

In the present invention, valves **31** and **32** are controlled by a controller **41.** The valves are preferably controlled in accordance with externally transmitted signals (not shown) received by a receiver **42** but may ultimately be controlled by any control system, including internal, mechanical, wired, or the like. The signals are preferably radio frequency (RF) signals transmitted by a remote control device **40** located external to the implanted gastric banding system. Power may be supplied to the receiver, the controller, and/or the valves either from an implanted power source **43** or from an induction coil **43** that receives power from a concentric coil external to the body, as described for instance for hearing aids in Baumann et al. (U.S. Patent 5,279,292).

The entirety of the remote gastric banding system **100** shown in Figure 3 may be laparoscopically implanted in the patient. Subsequent adjustment of the band can be simply, quickly, and painlessly performed using a remote control device to remotely inflate/deflate the inflatable portion **16** of the band. The entire system **100** may be removed from the patient if necessary. No permanent anatomical changes should be anticipated.

The remote control device **40** can be in the form of a typical television remote control, a personal computer interfaced device, or any other format. A unique identification code may be assigned to each remotely adjustable gastric band, so that access to and control of the device is restricted. This code may be a PIN code and may also act to prevent accidental adjustment of the band.

The system may be pressurized using a saline solution, or any other biocompatible fluid If desired, a concentrated saline solution may be used as the inflation medium, thereby allowing water from the patient's body to diffuse into the inflatable member **16** over time and further inflate the band. After repeated adjustments the reservoir **20** may be refilled through an access port (not shown) or replaced altogether. As a backup and safety measure, the system may also allow for inflation/deflation of inflatable member **16** by transdermal injection through a fill port (not shown) as in prior art gastric banding devices.

Because this system uses a pressurized reservoir rather than a mechanical pressurization means (*i.e*. a pump or screw), the present system is more energy-efficient than those disclosed in the existing remote-controlled adjustable gastric band systems of Klaiber or Forsell (U.S. Patents 5,938,669 and 6,210,347). Power is only required when operating the valves **31** and/or **32,** and then only for relatively short time intervals.

Alternative embodiments of the present invention may include means for measuring fluid flow through the valves **31** and/or **32,** such as a mass flowmeter, to ensure accuracy in adjusting the stoma when inflatable member **16** is inflated or deflated. Also, the controller **41** may be positioned external to the body. An alternate gastric band design might also be used, provided that the inflation medium remains a fluid.

A method of treating obesity using the remotely adjustable gastric banding system is disclosed herein for reference. The method includes implanting a gastric band, preferably laparoscopically, around the stomach of the patient to create a stoma; remotely transmitting control signals from outside of the patient to controller **41** of the gastric banding device inside of the patient; and opening and closing valve **31,** between pressurized reservoir **20** and inflatable chamber **16,** and/or valve **32,** between the inflatable chamber and outlet **23,** on the basis of the control signals received by controller **23** to increase or decrease the pressure in the inflatable chamber, thereby adjusting the inner circumference of the band to adjust the stoma size.

## Claims

1. A gastric banding apparatus for treatment of obesity in a patient, comprising:
a gastric band (10) suitable for laparoscopic placement around the stomach of the patient to create a stoma; said gastric band (10) having an inflatable chamber (16) for adjusting an inner circumference of the band;
a pressurized fluid reservoir (20) for providing fluid to inflate said inflatable chamber (16);
a first valve (31) between said pressurized fluid reservoir (20) and said inflatable chamber (16); a second valve (32) between said inflatable chamber (16) and the peritoneal cavity of the patient; or
an outlet (23) wherein said outlet (23) is a waste reservoir;
a controller (41) for actuating said first and second valves (31, 32) thereby increasing or decreasing the fluid volume in said inflatable chamber (16) to adjust the inner circumference of the band, said controller (41) being remotely controllable from outside of the patient.

2. The gastric banding apparatus according to claim 1, further comprising a remote control (40) for remotely transmitting control signals to the controller.

3. The gastric banding apparatus according to claim 1, further comprising a receiver (42) for receiving control signals wherein said controller (41) actuates said first and second valves (31, 32) in response to the received signals.

4. The gastric banding apparatus according to claim 1, further comprising a power source (43) for providing power to said controller (41), said first valve (31), and said second valve (32).

5. The gastric banding apparatus according to claim 4, wherein said power source (43) is an induction coil.

6. The gastric banding apparatus according to claim 4, wherein said power source (43) is a battery.

7. The gastric banding apparatus according to claim 4, wherein said power source (43) is a capacitor.

8. The gastric banding apparatus according to claim 7, wherein said capacitor is piezo-electrically charged.

9. The gastric banding apparatus according to claim 1, wherein said outlet (23) is a waste reservoir.

10. The gastric banding apparatus according to claim 9, wherein said waste reservoir is negatively pressurized.

11. The gastric banding apparatus according to claim 1, wherein said inflatable chamber (16) is substantially coextensive with an inner stomach-facing surface of said gastric band.

12. The gastric banding apparatus according to claim 11, wherein said inflatable chamber (16) does not wrinkle or fold when adjusted, thereby presenting a substantially smooth contour along said inner circumference.

13. The gastric banding apparatus according to claim 1, wherein said gastric band forms a smoothly surfaced circle.

14. The gastric banding apparatus according to claim 13, wherein said gastric band (10) is lockable in said smoothly surfaced circle.

15. The gastric banding apparatus according to claim 1, wherein the fluid in said pressurized fluid reservoir (20) is saline.

16. The gastric band apparatus according to claim 1, wherein a pressure in said pressurized fluid reservoir (20) remains greater than or equal to a pressure in said inflatable chamber (16).

17. The gastric band apparatus according to claim 1, wherein a pressure in said pressurized fluid reservoir (20) is greater than a pressure of said outlet (23).

## Patentansprüche

1. Magenbandvorrichtung zur Behandlung von Obesitas in einem Patienten mit:
einem Magenband (10), das für eine laparaskopische Anordnung um den Magen des Patienten, sodass ein Stoma gebildet wird, geeignet ist, wobei das Magenband (10) eine aufblasbare Kammer (16) zum Anpassen eines Innenumfangs des Bandes aufweist,
einem unter Druck gesetzten Fluidreservoir (20) zum Bereitstellen von Fluid zum Aufblasen der aufblasbaren Kammer (16),
einem ersten Ventil (31) zwischen dem unter Druck gesetzten Fluidreservoir (20) und der aufblasbaren Kammer (16), einem zweiten Ventil (32) zwischen der aufblasbaren Kammer (16) und der Bauchfellhöhle des Patienten, oder
einem Auslass (23), wobei der Auslass (23) ein Ablaufreservoir ist,
einer Steuerung (41) zum Betätigen der ersten und zweiten Ventile (31, 32), wodurch das Fluidvolumen in der aufblasbaren Kammer (16) erhöht oder verringert wird, sodass der Innenumfang des Bandes angepasst wird, wobei die Steuerung (41) von außerhalb des Patienten fernsteuerbar ist.

2. Magenbandvorrichtung nach Anspruch 1 darüber hinaus mit einer Fernsteuerung (40) zum Fernübertragen von Steuersignalen an die Steuerung.

3. Magenbandvorrichtung nach Anspruch 1 darüber hinaus mit einem Empfänger (42) zum Empfangen von Steuersignalen, wobei die Steuerung (41) die ersten und zweiten Ventile (31, 32) als Reaktion auf die empfangenen Signale betätigt.

4. Magenbandvorrichtung nach Anspruch 1 darüber hinaus mit einer Energiequelle (43) zum Bereitstellen von Energie an die Steuerung (41), das erste Ventil (31) und das zweite Ventil (32).

5. Magenbandvorrichtung nach Anspruch 4, wobei die Energiequelle (43) eine Induktionsspule ist.

6. Magenbandvorrichtung nach Anspruch 4, wobei die Energiequelle (43) eine Batterie ist.

7. Magenbandvorrichtung nach Anspruch 4, wobei die Energiequelle (43) eine Kapazität ist.

8. Magenbandvorrichtung nach Anspruch 7, wobei die Kapazität piezo-elektrisch geladen wird.

9. Magenbandvorrichtung nach Anspruch 1, wobei der Auslass (23) ein Ablaufreservoir ist.

10. Magenbandvorrichtung nach Anspruch 9, wobei das Ablaufreservoir mit negativem Druck beaufschlagt ist.

11. Magenbandvorrichtung nach Anspruch 1, wobei die aufblasbare Kammer (16) im Wesentlichen inhaltsgleich zu einer zum Magen zeigenden Innenfläche des Magenbandes ist.

12. Magenbandvorrichtung nach Anspruch 11, wobei die aufblasbare Kammer (16) nicht knittert oder sich faltet, wenn sie angepasst wird, wodurch sie eine im Wesentlichen glatte Kontur entlang des Innenumfangs darstellt.

13. Magenbandvorrichtung nach Anspruch 1, wobei das Magenband einen Kreis mit glatter Oberfläche bildet.

14. Magenbandvorrichtung nach Anspruch 13, wobei das Magenband (10) in dem Kreis mit glatter Oberfläche verriegelbar ist.

15. Magenbandvorrichtung nach Anspruch 1, wobei das Fluid in dem unter Druck gesetzten Fluidreservoir (20) salzhaltig ist.

16. Magenbandvorrichtung nach Anspruch 1, wobei ein Druck in dem unter Druck gesetzten Fluidreservoir (20) größer oder gleich einem Druck in der aufblasbaren Kammer (16) verbleibt.

17. Magenbandvorrichtung nach Anspruch 1, wobei ein Druck in dem unter Druck gesetzten Fluidreservoir (20) größer ist als ein Druck des Auslasses (23).

## Revendications

1. Appareil de bandage gastrique pour le traitement de l'obésité chez un patient, comprenant :
une bande gastrique (10) appropriée pour une mise en place laparoscopique autour de l'estomac du patient afin de créer une stomie ; ladite bande gastrique (10) comportant une chambre gonflable (16) pour ajuster une circonférence interne de la bande ;
un réservoir de fluide sous pression (20) pour fournir du fluide afin de gonfler ladite chambre gonflable (16) ;
une première valve (31) entre ledit réservoir de fluide sous pression (20) et ladite chambre gonflable (16) ; une deuxième valve (32) entre ladite chambre gonflable (16) et la cavité péritonéale du patient ; ou
une sortie (23), dans lequel ladite sortie (23) est un réservoir de déchets ;
un contrôleur (41) pour actionner lesdites première et deuxième valves (31, 32), augmentant ou diminuant ainsi le volume de fluide dans ladite chambre gonflable (16) afin d'ajuster la circonférence interne de la bande, ledit contrôleur (41) pouvant être contrôlé à distance depuis l'extérieur du patient.

2. Appareil de bandage gastrique selon la revendication 1, comprenant en outre une commande à distance (40) pour transmettre à distance des signaux de commande au contrôleur.

3. Appareil de bandage gastrique selon la revendication 1, comprenant en outre un récepteur (42) pour recevoir des signaux de commande, dans lequel ledit contrôleur (41) actionne lesdites première et deuxième valves (31, 32) en réponse aux signaux reçus.

4. Appareil de bandage gastrique selon la revendication 1, comprenant en outre une source d'énergie (43) pour fournir de l'énergie audit contrôleur (41), à ladite première valve (31) et à ladite deuxième valve (32).

5. Appareil de bandage gastrique selon la revendication 4, dans lequel ladite source d'énergie (43) est une bobine d'induction.

6. Appareil de bandage gastrique selon la revendication 4, dans lequel ladite source d'énergie (43) est une batterie.

7. Appareil de bandage gastrique selon la revendication 4, dans lequel ladite source d'énergie (43) est un condensateur.

8. Appareil de bandage gastrique selon la revendication 7, dans lequel ledit condensateur est chargé de manière piézoélectrique.

9. Appareil de bandage gastrique selon la revendication 1, dans lequel ladite sortie (23) est un réservoir de déchets.

10. Appareil de bandage gastrique selon la revendication 9, dans lequel ledit réservoir de déchets est sous pression négative.

11. Appareil de bandage gastrique selon la revendication 1, dans lequel ladite chambre gonflable (16) est sensiblement co-extensive avec une surface interne orientée vers l'estomac de ladite bande gastrique.

12. Appareil de bandage gastrique selon la revendication 11, dans lequel ladite chambre gonflable (16) ne fronce pas ou ne fait pas de pli lorsqu'elle est ajustée, présentant ainsi un contour sensiblement lisse le long de ladite circonférence interne.

13. Appareil de bandage gastrique selon la revendication 1, dans lequel ladite bande gastrique forme un cercle à surface lisse.

14. Appareil de bandage gastrique selon la revendication 13, dans lequel ladite bande gastrique (10) peut se verrouiller dans ledit cercle à surface lisse.

15. Appareil de bandage gastrique selon la revendication 1, dans lequel le fluide dans le réservoir de fluide sous pression (20) est une solution saline.

16. Appareil de bandage gastrique selon la revendication 1, dans lequel une pression dans ledit réservoir de fluide sous pression (20) reste supérieure ou égale à une pression dans ladite chambre gonflable (16).

17. Appareil de bandage gastrique selon la revendication 1, dans lequel une pression dans ledit réservoir de fluide sous pression (20) est supérieure à une pression de ladite sortie (23).
